# EUROPÄISCHE PATENTSCHRIFT

(11) **EP 1 894 209 B1**
(45) Veröffentlichungstag und Bekanntmachung des Hinweises auf die Patenterteilung: **24.08.2011**
(21) Anmeldenummer: 05776644.6
(22) Anmeldetag: 10.08.2005
(51) Int. Cl.: G21K 1/04

(54) **KOLLIMATOR UND SCANVORRICHTUNG**
COLLIMATOR AND SCANNING DEVICE
COLLIMATEUR ET SCANNER

(30) Priorität: 06.05.2005 EP 05009871
(43) Veröffentlichungstag der Anmeldung: 05.03.2008
(73) Patentinhaber: Deutsches Krebsforschungszentrum (DKFZ), 69120 Heidelberg (DE)
(72) Erfinder: ECHNER, Gernot, 69257 Wiesenbach (DE)
(74) Vertreter: Weber, Walter
(86) Internationale Anmeldenummer: PCT/EP2005/008659
(87) Internationale Veröffentlichungsnummer: WO 2006/119796

(56) Entgegenhaltungen:
- EP-A- 0 382 560
- EP-A- 1 367 604
- DE-A1- 1 564 765
- DE-B- 1 037 035
- FR-A- 1 605 155
- FR-A- 2 524 690
- FR-A5- 2 113 337
- US-A- 2 675 486

## Beschreibung

Die Erfindung betrifft einen Kollimator und Scannvorrichtung, wobei der Kollimator zum Begrenzen eines Bündels energiereicher Strahlen dient, das von einer im wesentlichen punktförmigen Strahlungsquelle ausgehend auf ein Behandlungsobjekt gerichtet ist und insbesondere der stereotaktischen Konformationsbestrahlung von Tumoren dient, wobei der Kollimator eine Blende aufweist und die Scannvorrichtung die Fläche eines Behandlungsobjekts aus einer Raumwinkelausrichtung mittels der durch die Blende begrenzten Strahlen abscannt.

Kollimatoren zum Begrenzen eines Bündels energiereicher Strahlen werden zu Diagnosezwecken und zur Behandlung insbesondere von Tumoren eingesetzt. Dabei dienen die Kollimatoren zur Begrenzung der Strahlen, damit neben dem Diagnose- bzw. Behandlungsbereich liegendes gesundes Gewebe möglichst gut vor Strahlen geschützt wird, um Schäden zu vermeiden oder auf ein Minimum zu reduzieren.

Ursprünglich waren die Kollimatoren derart beschaffen, daß mit ihnen ein Bestrahlungsfeld lediglich der Größe nach begrenzbar war. Wurden lediglich Röntgenstrahlen zur Bilderzeugung eingesetzt, so ergaben sich daraus keine schwerwiegende Beeinträchtigung des Patienten. Erst die therapeutische Bestrahlung mit energiereichen Strahlen - bei der beispielsweise Tumorgewebe zerstört werden soll - führten im Überstrahlungsbereich, also außerhalb des zu bestrahlenden kranken Gewebes zu einer Schädigung von gesundem Gewebe. Solche Überstrahlungsbereiche kamen zum einen dadurch zustande, daß die Kontur des kranken Gewebes durch die Kollimatoren nicht nachgebildet wurde, zum anderen dadurch, daß an den Begrenzungen des Bestrahlungsbereichs Halbschatten entstanden, da dort, insbesondere bei großen Bestrahlungsfeldern, nicht die volle Stärke des Abschirmungsmaterials zur Verfügung stand, weil es nicht parallel zu den Strahlen ausgerichtet war.

Ein Beispiel für einen solchen Kollimator älterer Bauart ist die US 2,675,486. Diese Schrift betrifft einen Kollimator zur Begrenzung energiereicher Strahlen, der vier Strahlbegrenzungsblöcke aufweist, welche mittels aneinandergrenzenden Seitenflächen derart in einer Ebene verschiebbar sind, daß damit eine quadratische Strahlbegrenzung unterschiedlicher Größen einstellbar ist. Da Tumore keine quadratische, sondern eher eine abgerundete Form haben, ergibt sich ein großer Überstrahlungsbereich an den Ecken. Außerdem kam es bei großen Bestrahlungsfeldern zu großen Halbschattenbereichen, da die Blockbegrenzungen nicht mehr parallel zum divergierenden Strahlengang verlaufen.

Die Fachwelt bemühte sich deshalb darum, diese Probleme zu entschärfen:
Von einem Kollimator der vorgenannten Art ausgehend schlägt die DE 20 53 089 A 1 daher für den vom Erfindungsgegenstand nicht ganz entfernt liegenden Bereich der Röntgenbilderzeugung vor, Abschirmungskörper in Form von aneinandergrenzenden Dreiecken vorzusehen, um ein annähernd kreisförmiges - also der Form eines Bestrahlungsbereichs eher entsprechendes - Bestrahlungsfeld zu erzielen, so daß eine durch die Ecken der vorgenannten quadratischen Strahlbegrenzung hervorgerufene, in etwa dreißigprozentige Überstrahlung vermieden wird. Die verbleibende Überstrahlung sowie eine Halbschattenbildung stellen hier jedoch kein gravierendes Problem dar, da es lediglich um Röntgenstrahlen zur Bilderzeugung, jedoch nicht um eine therapierende Bestrahlung mit wesentlich energiereicheren Strahlen geht.

Die DE 15 89 432 A1 schlägt für den Einsatz der hier in Frage stehenden ionisierenden, also für die Tumorbehandlung geeigneten, energiereichen Strahlen einen Kollimator vor, bei dem aneinandergrenzende keilförmige Strahlungsabschirmungskörper in einer Ebene derart verschiebbar sind, daß sechseckige, achteckige oder rechteckige Öffnungen zusammengestellt werden können. Dieser Kollimator bildet jedoch eine Tumorform nur sehr unzureichend nach und gegen einen Halbschatten sind keine Vorkehrungen getroffen. Bei großen Bestrahlungsfeldern, bei denen der Strahlengang sehr schräg zu der Begrenzung des Abschirmungsmaterials verläuft, tritt ein großer Halbschatten auf.

Auch die DE 10 37 035 B geht von einem Kollimator der Art der erstgenannten Schrift aus und sieht für energiereiche Therapiestrahlen vor, die vier Strahlbegrenzungsblöcke entlang einer schrägen Linie in zwei Teile zu teilen, wobei die Linie zu der Stelle verläuft, an der sich die Innen- und Endfläche (also die an den nächsten Block grenzende Fläche) treffen. Dadurch erhält man von jedem Block ein Haupt- und ein Nebenteil, welche gegenseitig verschiebbar sind. Dadurch wird die Bildung verschiedener Umrisse ermöglicht, was ebenfalls die Überstrahlung gegenüber einer quadratischen Strahlbegrenzung reduziert. Allerdings ist die Nachbildung der Form eines Tumors oder eines sonstigen zu bestrahlenden Bereichs nur sehr unzureichend gelöst und das Halbschattenproblem wird nicht gelöst.

Die Lösung des Halbschattenproblems lehrt schließlich die DE 15 64 765 A1. Diese Schrift geht ebenfalls von einem Kollimator der Art der erstgenannten Schrift mit vier aneinandergrenzenden, in einer Ebene verschiebbaren Strahlungsbegrenzungsblöcken aus. Sie setzt sich zum Ziel, ein scharf abgegrenztes Feld, also ein Feld ohne Halbschatten zu erzielen. Es wird vorgeschlagen, zu diesem Zweck die Blöcke derart auszugestalten und schwenkbar zu lagern, daß die die Strahlungsbegrenzung bildenden Stirnflächen bei jeder Einstellung auf die Strahlenquelle gerichtet sind. Dadurch schirmt immer das Material der Blöcke in voller Stärke die Strahlung ab. Allerdings lassen sich mit diesem Kollimator wiederum nur quadratische Bestrahlungsfelder bilden, so daß hier wieder große Überstrahlungsbereiche an den Ecken in Kauf genommen werden mußten.

Sowohl das Problem der Überstrahlungsbereiche, als auch das Halbschattenproblem geht die FR 2 524 690 an. Diese Schrift sieht zur Halbschattenverhinderung bzw. -verminderung vor, aneinandergrenzende, in einer Ebene verschiebbare Platten in mehreren Ebenen anzuordnen, so daß eine gestufte, pyramidenstumpfförmige Strahlbegrenzungsöffnung entsteht. Auf diese Weise wird der Halbschatten minimiert. Er tritt nur noch in dem Bereich auf, in dem die Strahlen die Stufenform durchqueren. Je größer die zu begrenzende Fläche wird, je größer wird allerdings dieser Stufenbereich des trotz dieser Maßnahme verbleibenden Halbschattens. Ein weiterer Nachteil dieses Lösungsvorschlags besteht darin, daß als Bestrahlungsfeldbegrenzung nur Vielecke - in Abhängigkeit von der Anzahl der Platten - gebildet werden können und eine Formgebung in der wirklichen Kontur eines Tumors nicht möglich ist.

Aus der EP 1 367 604 A1 ist eine Vorrichtung zum Konzentrieren eines Röntgenstrahls zu einem Mikroröntgenstrahl bekannt, wobei die Konzentration durch Reflexion an spiegelnden Innenflächen einer Kapillarröhre erfolgt. Diese Kapillarröhre wird durch Verschieben von konzentrisch angeordneten Stangensegmenten gebildet, welche mittels Schrauben verschieb- und justierbar sind. Dies Vorrichtung läßt jedoch nur eine sehr begrenzte punktuelle Bestrahlung zu. Außerdem ist der Effekt der Reflexion an spiegelnden Innenflächen nicht für Therapiestrahlen geeignet, die im Megavoltbereich liegen.

Um Tumorformen besser nachbilden und die Überstrahlung auf ein Minimum reduzieren zu können, ging man schließlich dazu über, wechselbare feste Kollimatoren zu verwenden. Dabei wurde die Tumorform aus verschiedenen räumlichen Richtungen erfaßt und es wurden für jede Bestrahlung mehrere feste Kollimatoren hergestellt, die dann für die Bestrahlung aus den verschiedenen Richtungen eingesetzt wurden. Dabei hatte man den Vorteil exakter Formgebung und auch die Möglichkeit, die Begrenzungen exakt dem Strahlengang anzupassen, wodurch kein Halbschatten mehr auftritt. Der Nachteil bestand jedoch in dem umständlichen Verfahren mit ständigem Kollimatorwechsel, der viel Zeit an wertvollen Geräten beanspruchte sowie in dem Aufwand für die Herstellung vieler Kollimatoren für jede Bestrahlung, die danach nicht mehr zu gebrauchen waren, da sie zur Anwendung für einen Patienten bestimmt und auch für diesen nur in einem zeitlich begrenzten Rahmen verwendbar waren. Letzteres aufgrund des Umstandes, daß sich die Form des Tumors durch Wachstum, Rückbildung oder Formveränderung ständig ändert.

Um diesen Aufwand zu verringern, wurden Multileafkollimatoren geschaffen, bei denen mit einer Vielzahl von schmalen, eng aneinanderliegenden Leafs (also Blendenblättern) die Gestalt des Tumors mittels Stellbewegungen der Leafs nachgebildet werden konnte. Zunächst hatten diese Multileafkollimatoren zwar den Vorteil, daß sehr schnell fast beliebige Formen einstellbar waren, sie hatten jedoch den Nachteil einer sehr aufwendigen Mechanik mit Stellmitteln für jedes Leaf und den weiteren Nachteil, daß an jeder Begrenzung des Bestrahlungsfeldes durch ein Leaf, je nach Entfernung desselben von der Achse des Strahlengangs ein mehr oder weniger großer Halbschatten auftrat.

Zur Vermeidung solcher Halbschatten wurde von der EP 1 153 397 B1 vorgeschlagen, die Leafs mit verstellbaren Vorderkanten auszustatten, wobei eine Mechanik sie immer parallel zum Strahlengang stellt. Dies hat allerdings eine noch aufwendigere Mechanik des Multileafkollimators zur Folge.

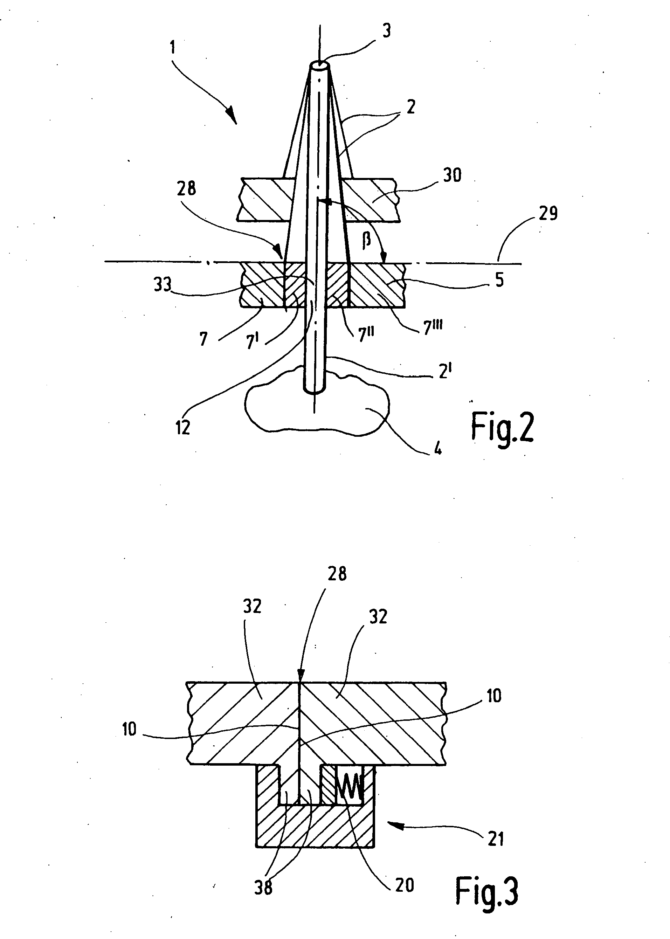

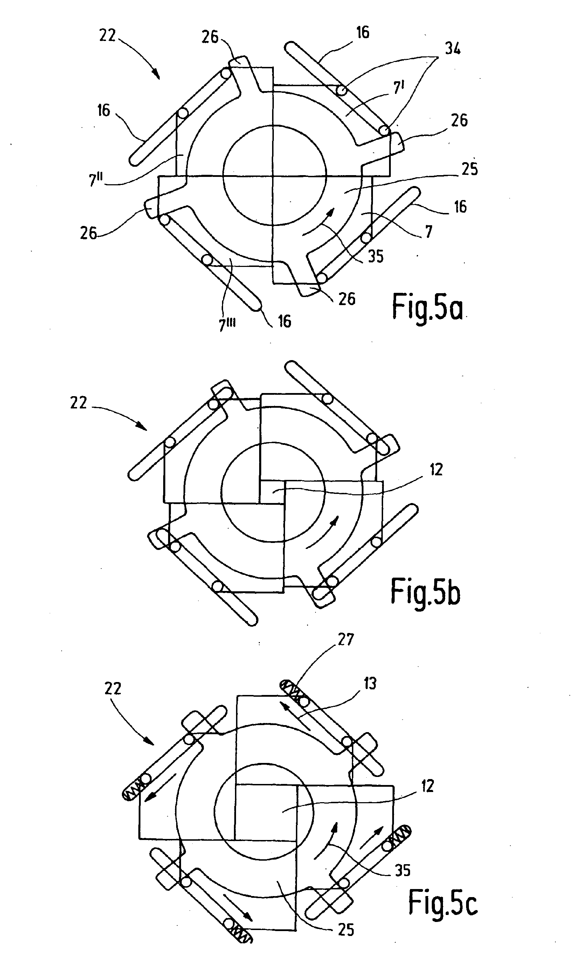

Um diese aufwendige Mechanik zu vermeiden und in der Formung einer zu bestrahlenden Fläche noch flexibler zu sein, wurde schließlich von der DE 199 22 656 A1 eine Scannvorrichtung mit einer Kollimatoröffnung vorgeschlagen, die derart klein ausgebildet ist, daß das zu bestrahlende Objekt bezüglich seiner zu bestrahlenden Bereiche mit ausreichender Genauigkeit bestrahlbar ist (Fig. 3). Beim vorgenannten Vorschlag führt eine kleine Kollimatoröffnung zwar zu einer großen Genauigkeit, jedoch zu einem langsameren Abscannen - bei einer großen Blendenöffnung kann schneller abgescannt werden, es ist jedoch die erforderliche Genauigkeit nicht möglich. Auch die Verwendung von Mehrlochplatten zur Erzeugung eines Bündels aus mehreren Scannstrahlen (Fig. 5 und 5a) führte dabei noch nicht zu einer befriedigenden Bestrahlungszeitverkürzung. Die Mehrlochplatte war bezüglich der Bestrahlungsfläche festgelegt und zur genauen Bestrahlung der Randbereiche mußten noch kleinere Blendenöffnungen verwendet, also die Platten gewechselt werden.

Um die Abscanngeschwindigkeit zu erhöhen, ohne auf eine hohe Genauigkeit verzichten zu müssen, wurde schließlich von der DE 101 57 523 C1 vorgeschlagen, einen Kollimator und eine Scannvorrichtung (dort beides zusammen "Kollimator" genannt) der eingangs genannten Art mit mehreren verschieden großen Kollimatoröffnungen vorzusehen, die wahlweise in den Strahlengang bringbar sind. Dies erfolgte vorzugsweise mittels eines revolverähnlichen Mechanismus, der eine runde Platte mit den verschieden großen Öffnungen dreht. Bei den heute für die Therapie gebräuchlichen energiereichen Strahlen ist jedoch zur Abschirmung eine Materialstärke von 6 bis 10 cm erforderlich. Auf diese Weise erhält man entweder einen Kollimator mit sehr hohem Gewicht, oder man muß sich auf wenige, beispielsweise drei Öffnungsgrößen beschränken. Aber selbst bei einer solchen Beschränkung ist es erforderlich, die nicht verwendeten Öffnungen abzudecken, damit nicht Bereiche entstehen, welche nur mit unzureichender Materialdicke abgeschirmt sind. Es ist also neben der Platte mit den Öffnungen noch eine Abschirmplatte erforderlich, welche ebenfalls mehrere Zentimeter Stärke aufweisen muß. Aus diesem Grund wird der Kollimator relativ schwer, was den Aufwand an Führungen und Antrieben entsprechend vergrößert. Ein weiterer Nachteil dieses Kollimators besteht darin, daß aus den oben genannten Gründen nur wenige der festen Kollimatoröffnungen verfügbar sind, wodurch die Variabilität der Strahlbegrenzung stark limitiert ist. Insbesondere ist es auch aus den oben genannten Gründen nicht möglich, große Öffnungen verschiedener Durchmesser vorzusehen, mit denen zunächst ein Bereich der zu bestrahlenden Fläche behandelt werden kann, der möglichst groß ist, um danach noch die Randbereiche mit abgestuft feineren Strahlenbündeln zu behandeln. Da die Verweilzeit einer Strahlenapplikation für jeden Punkt einer Fläche mehrere Sekunden ausmacht, bedeutet dies, daß ein Abscannen einer Fläche mit festgelegten Größen von Strahlenbündeln zeitaufwendiger ist als mit optimalen einstellbaren Größen. Dies ist insbesondere dann der Fall, wenn die Strahlenbündel schmäler sind, als dies bezüglich der Bestrahlungsfläche möglich wäre. Dadurch wird die Gesamtbehandlungsdauer verlängert. Dies ist nicht nur für den Patienten, der fixiert werden muß, unangenehm, sondern senkt auch die Anzahl der an einem Gerät möglichen Behandlungen, was in Anbetracht hoher Anschaffungs- und Betriebskosten solcher Geräte von hoher wirtschaftlicher Bedeutung ist. Außerdem ist die Genauigkeit der Randbereicherfassung begrenzt, was in Bereichen wie angrenzende Nerven kritisch ist.

Die EP 0 382 560 A1 offenbart zwar als strahlbegrenzendes Mittel eine Irisblende und erwähnt eine Bestrahlung durch ein "Scannen". Es handelt sich jedoch nicht um eine Scannbewegung der in der DE 199 22 656 A1 oder der DE 101 57 523 C1 genannten Art, bei der eine Fläche durch die Scannbewegung eines begrenzten Strahls ihre Strahlenapplikation erhält. Diese Applikationen der jeweiligen Flächen des Behandlungsobjekts erfolgen dann nacheinander aus verschiedenen Raumwinkeln, indem eine Gantry mit Strahlenquelle, Strahlenbegrenzung und Scannvorrichtung den Patienten umkreist. Bei der EP 0 382 560 A1 wird viel mehr die vorgenannte Umkreisung des zu bestrahlenden Bereichs als "scannen" bezeichnet. Die Applikation aus einer Richtung erfolgt jedoch nicht durch das Abscannen einer Fläche, also derart, wie "scannen" in der Technik üblicherweise verstanden wird. Vielmehr wird die jeweils aus einer Richtung auf eine Behandlungsfläche zu applizierende Bestrahlung ungefähr mit der Irisblende eingestellt, wie dies in den Figuren 2 bis 5 dargestellt und in der Beschreibung der EP 0 382 560 A1 beschrieben ist. Diese Flächen sind dann aber immer Vielecke entsprechend der Blendenblätter der Irisblende, also annähernd Kreise. Eine derart grobe Erfassung einer Fläche kann eine Tumorform nicht nachbilden, sie führt daher zur Zerstörung von mitbestrahltem, gesundem Gewebe. Damit ist aber der Vorschlag der EP 0 382 560 A1 mit Nachteilen behaftet, die heute nicht mehr akzeptabel sind und durch die technische Entwicklung - wie von der DE 199 22 656 A1 und der DE 10157 523 C1 vorgeschlagen - bereits überwunden wurden.

Die Erfindung geht daher von einer Scannvorrichtung aus, wie sie in der DE 101 57 523 C1 offenbart wurde. Diese Schrift entspricht dem eingangs erwähnten Kollimator, mit dem die Fläche eines Behandlungsobjekts abgescannt wird.

Der Erfindung liegt daher die Aufgabe zugrunde, einen Kollimator und eine Scannvorrichtung der eingangs und vorgenannten Art derart auszubilden, daß bei hoher Abschirmung und geringer Bauhöhe die Bestrahlungszeit verkürzt und die zu bestrahlende Fläche genauer erfaßt werden kann.

Die Aufgabe wird erfindungsgemäß dadurch gelöst, daß der Kollimator als strahlbegrenzendes Mittel eine Irisblende mit mindestens drei Blendenblätter aufweist, die gleiche Winkel einschließende, aneinanderliegende Seitenflächen aufweisen, wobei die Blendenblätter eine strahlbegrenzende Öffnung dadurch freigeben, daß mittels eines Antriebs eine Schiebebewegung entlang der Seitenflächen durch eine höchstens um eines verminderte Anzahl von Blendenblättern stattfindet.

Der Grundgedanke der Erfindung besteht darin, daß eine einstellbare Irisblende schneller und mit variabler Blendenöffnung auf die erforderliche Größe zu bringen ist, als dies durch den Wechsel von festen Blenden möglich ist. Gegenüber Blendenplatten mit mehreren in den Strahlengang bringbaren Öffnungen weist sie dagegen sowohl ein geringeres Gewicht, als auch eine höhere Flexibilität bezüglich der einstellbaren Blendenöffnungsweite auf.

Der Aufbau ist relativ einfach und die für die Einstellung der Blendenöffnung erforderliche Stellbewegung läßt sich mit einfachen mechanischen oder elektronischen Mitteln erzielen. Die kompakte Bauweise erlaubt es auch, den mit der Irisblende ausgestatteten Kollimator durch entsprechende Vorrichtungen in die erforderlichen Positionen zu bringen. Dies ist im Bereich der therapierenden Bestrahlung erforderlich für das Abscannen einer Fläche sowie für eine Bestrahlung, die aus unterschiedlichen Raumwinkeln auf das Behandlungsobjekt gerichtet ist.

Die Irisblende ist derart ausgebildet, daß keinerlei Überlappung von Blendenblätter mehr erforderlich ist. Dies wird erreicht, indem alle Blendenblätter in einer Ebene liegen und deren Seitenflächen aneinanderliegen. Erst nach einer Schiebebewegung der Blendenblätter zur Herstellung der Blendenöffnung liegen kurze vordere Teilbereiche der Seitenflächen der Blendenblätter frei, um die auf diese Weise gebildete strahlbegrenzende Blendenöffnung zu begrenzen. Auf diese Weise wird es möglich, Irisblenden auch im Bereich sehr energiereicher Strahlen einzusetzen, wobei trotz der für die Strahlenabschirmung erforderlichen Dicke der Blendenblätter eine handhabbare Bauhöhe eingehalten werden kann. Da keine Überlappungsbereiche erforderlich sind, ist das Gewicht gegenüber einer üblichen Irisblendenkonstruktion geringer. Das Gewicht, welches im Wesentlichen durch das abschirmende Material bestimmt wird, entspricht ungefähr dem Gewicht von Festblenden.

Da mittels des Kollimators und der Scannvorrichtung ein Abscannen einer Fläche vorgenommen wird, ist eine Scannvorrichtung vorgesehen, welche ein Behandlungsobjekt mittels der durch die Irisblende begrenzten Strahlen abscannt. Eine derartige Scannvorrichtung ist aus der deutschen Patentschrift DE 101 57 523 C1 bekannt. In diesem Fall tritt die Irisblende an die Stelle der dort offenbarten Kollimatoröffnungen mit festen Größen, die dort wahlweise in der erforderlichen Größe in den Strahlengang gebracht werden müssen. Alle übrigen in dieser Schrift offenbarten Merkmale lassen sich auf den Kollimator mit der Irisblende entsprechend übertragen, insbesondere die Mechanik um Strahlenquelle und Irisblende in der erforderlichen Scannbewegung zu führen. Deren Beschreibung wird daher durch diesen Verweis ersetzt. Eine andere Möglichkeit einer solchen Scannvorrichtung besteht darin, daß sich Strahlenquelle und Irisblende an einem Roboterarm befinden, der um das Behandlungsobjekt bewegbar ist. Derartige Roboterarme sind bekannt. Sie finden insbesondere in der automatisierten Fertigung bereits zahlreiche Verwendung, so daß auch auf deren nähere Beschreibung verzichtet werden kann.

Bei einer Behandlung kann man die Öffnung zuerst größer lassen, um eine Fläche abzuscannen und dann klein zu machen und mit einer Scannbewegung beliebige Formen, wie beispielsweise die unregelmäßigen Randbereiche der Fläche eines Behandlungsobjekts, abzuscannen.

Strahlenquelle, Kollimator und Scannvorrichtung können auch mittels einer Gantry in verschiedne Raumwinkelausrichtungen der durch den Kollimator begrenzten Strahlen zum Behandlungsobjekt bringbar sein. Selbstverständlich ist auch die oben genannte Scannvorrichtung in einer solchen Gantry aufgehängt, um ein zu bestrahlendes räumliches Gebilde von verschiedenen Seiten abzuscannen. Derartige Gantrys finden bereits in Bestrahlungsgeräten üblicherweise Verwendung, insbesondere im Zusammenhang mit Multileafkollimatoren, welche die zu bestrahlende Fläche mittels einer aufwendigen Mechanik nachbilden.

Der Zweck der Erfindung besteht insbesondere darin, eine Abschirmungsfähigkeit für besonders energiereiche Strahlen bei geringer Bauhöhe einer Irisblende verfügbar zu machen. Für solche Anwendungsbereiche sollte die Abschirmungsfähigkeit für energiereiche Strahlen bezüglich einer Strahlungsquelle im Megavoltbereich ausgelegt sein. Dies betrifft dann hauptsächlich den Einsatzbereich der Strahlentherapie, da beispielsweise für die Zerstörung von Tumorgeweben solche energiereichen Strahlen erforderlich sind. Für diesen Zweck sollte die Dicke der Blendenblätter zwischen 6 und 10 cm liegen, wobei übliches Blendenblattmaterial vorausgesetzt wird, das beispielsweise eine Wolframlegierung sein kann.

Prinzipiell ist es möglich, daß ein Blendenblatt fest angeordnet ist und sich die anderen Blendenblätter zur Bildung der Öffnung um gleiche Teilstücke entlang der Kante eines angrenzenden Blendenblattes verschieben. Damit jedoch der Mittelpunkt der Blendenöffnung unabhängig von deren Größe immer an der gleichen Stelle ist, ist es zweckmäßig, wenn eine Schiebebewegung aller Blendenblätter um gleiche Stellwege erfolgt, so daß nach der Stellbewegung die Öffnung durch Teilbereiche der Seitenflächen gebildet wird, die gleiche Entfernungen vom Mittelpunkt aufweisen. Bei dieser Ausgestaltung bleibt die optische Achse unabhängig von der Öffnungsbewegung der Blendenblätter der Irisblende immer an derselben Stelle.

Was bezüglich der Lagerung der Blendenblätter erforderlich ist, hängt davon ab, ob alle Blendenblätter eine Schiebebewegung vollziehen und wie viele Blendenblätter vorhanden sind. Bei lediglich drei Blendenblättern, bei denen eines fest ist, reicht zur Führung der beiden verschiebbaren Blendenblätter eine sichere Anlage an den Seitenflächen des feststehenden Blendenblattes aus. Insbesondere wenn alle Blendenblätter verschiebbar sind, erfordert ein eindeutiger Bewegungsverlauf, daß die Blendenblätter mittels in Richtung der Schiebebewegung verlaufenden Linearführungen gelagert sind. Bezüglich des Verlaufs der Linearführungen muß der genaue Verlauf der Bewegung eines jeden Blendenblattes berücksichtigt werden. Dies ist aus der Figurenbeschreibung noch näher ersichtlich. Beispielsweise ergibt sich für eine vierblättrige Blende, daß die Linearführungen in einem 45°-Winkel zu den Seitenflächen verlaufen müssen, die an die anderen Blendenblätter anliegen.

Eine besonders zweckmäßige Ausgestaltung besteht darin, daß die Irisblende vier Blendenblätter aufweist. Dies ergibt eine quadratische Öffnung, welche in einer Scannbewegung derart über die Fläche eines Behandlungsobjekts geführt werden kann, daß am Ende des Scannvorgangs die Bestrahlungsdauer auf der gesamten bestrahlten Fläche exakt gleich ist.

Eine Irisblende mit vier Blendenblättern kann auch so ausgestaltet werden, daß die jeweilige, die Öffnung bildende Seitenfläche an ihrem inneren Ende in einen nasenartig auskragenden, einen Viertelkreis bildenden Kreisbogen übergeht, der dann den Abschluß dieser Seitenfläche bildet. Werden dann die vier Blendenblätter derart zusammengefügt, daß die Kreisbögen unmittelbar aneinanderliegen, so entsteht eine runde Öffnung. Eine solche ist insbesondere dann zweckmäßig, wenn für ein Scannen oder eine punktuelle Bestrahlung ein Einzelstrahl mit einem sehr kleinen Durchmesser benötigt wird. Werden derartige Blendenblätter weiter geöffnet, so entsteht eine quadratische Öffnung mit runden Ecken, wobei verschiedene Größen möglich sind. Diese kann dann in der oben genannten Art und Weise zum Scannen eingesetzt werden. Auf diese Weise läßt sich mit relativ großen quadratischen Öffnungen der Großteil einer Fläche abscannen und es ist möglich, mit der runden kleinen Öffnung einen sehr feinen Strahl zu erzielen, mit dem unregelmäßige Randbereiche, für die die quadratische Öffnung zu großflächig ist, noch abgescannt werden können.

Alternativ kann die Irisblende dafür eingerichtet sein, daß ein möglichst runder Strahl geformt wird. Dafür sind dann mindestens sechs Blendenblätter zweckmäßig, wobei sich mit der Anzahl natürlich die Annäherung an die Kreisform immer mehr verbessert. Dies ermöglicht es, eine große Öffnung zu bilden, wie sie beispielsweise für eine Röntgenbestrahlung zum Zweck der Diagnose erforderlich ist oder man kann mit energiereicheren Strahlen einen runden Tumor, wie beispielsweise einen Gehirntumor, bestrahlen.

Bei dem erfindungsgemäßen Kollimator muß gewährleistet sein, daß die Seitenflächen der Blendenblätter der Irisblende exakt aufeinanderliegen. Sie müssen daher exakt plan sein und dürfen keine Oberflächenrauheit aufweisen. Dazu ist gegebenenfalls eine Feinstbearbeitung, wie zum Beispiel Schleifen oder Läppen erforderlich. Weiterhin ist notwendig, daß die Seitenflächen fest aneinanderliegen, wozu vorgeschlagen wird, daß Kraftbeaufschlagungen vorgesehen sind, die die Seitenflächen der Blendenblätter gegeneinander drücken. Beispielsweise kann vorgesehen sein, daß auf die Blendenblätter Federn wirken, die die Kraftbeaufschlagungen bewirken. Durch derartige Kraftbeaufschlagungen läßt sich eine noch bessere Flächenanlage als durch präzise Führungen erzielen, da Führungen immer ein kleines Spiel aufweisen müssen.

Eine weitere Möglichkeit für ein gutes Aneinanderliegen der Seitenflächen besteht darin, daß diese in ihren nicht zur Bildung der Blendenöffnung dienenden aneinanderliegenden Bereichen gemeinsame Führungen aufweisen, in denen die Seitenflächen der aneinandergrenzenden Blendenblätter gegenseitig verschiebbar sind. Auch eine derartige gemeinsame Führung zweier Seitenflächen aneinandergrenzender Blendenblätter kann eine Kraftbeaufschlagung durch Federn zum Zweck einer präzisen Flächenanlage der Seitenflächen beinhalten.

Die Schiebebewegung der Blendenblätter wird dadurch erreicht, daß mindestens ein Blendenblatt angetrieben wird. Dies reicht insbesondere bei der bereits erwähnten dreiblättrigen Irisblende aus. Sind mehr Blendenblätter vorhanden, so kann beispielsweise jedes zweite Blendenblatt angetrieben werden und die anderen Blendenblätter bewegen sich durch die dabei erzielte Kraftübertragung mit. Für einen möglichst reibungsfreien und exakten Bewegungsablauf der Öffnungsbewegung ist es jedoch zweckmäßig, wenn alle Blendenblätter simultan angetrieben werden.

Ein solcher simultaner Antrieb aller Blendenblätter kann auf unterschiedliche Weise erfolgen. Beispielsweise ist es möglich, daß für jedes Blendenblatt ein Antrieb vorgesehen ist, wobei durch eine elektronische Steuerung eine simultane Bewegung erfolgt. Diese muß allerdings sehr präzise sein, da ein ungleichmäßiger Antrieb dazu führen würde, daß sich die Blendenblätter gegenseitig verkeilen. Eine weitere Möglichkeit sieht vor, daß ein Antrieb über eine Mechanik alle Blendenblätter simultan antreibt. Derartige Mechaniken können auf unterschiedlichste Weise ausgebildet werden. So können beispielsweise Spindel- oder Schneckentriebe vorgesehen sein, die über ein Getriebe simultan bewegt werden. Eine weitere Möglichkeit besteht darin, daß die Mechanik eine um den Mittelpunkt der Blendenöffnung drehbare Kurvenscheibe aufweist, wobei natürlich eine Öffnung in der Mitte der Kurvenscheibe erforderlich ist, die einen Strahldurchtritt des größtmöglichen Strahls zuläßt. Auf dieser Kurvenscheibe sind dann spiralförmig Stellkurven angeordnet, welche die Blendenblätter betätigen. Bei den Stellkurven kann es sich um Nuten oder Erhöhungen handeln, welche die Blendenblätter durch Elemente verstellen, die an diesen angeordnet sind und auf den Stellkurven gleiten.

Eine weitere Möglichkeit des Aufbaus einer solchen Mechanik sieht vor, daß ein um den Mittelpunkte der Blendenöffnung drehbares Stellorgan vorgesehen ist, das auf jedes Blendenblatt mit einem Stellarm wirkt. Dann ist es natürlich zweckmäßig, wenn eine derartige Schiebebewegung mittels der Stellarme auch eine Rückstellung beinhaltet. Solche kann beispielsweise durch Rückstellfedern, die der Stellbewegung entgegenwirken, erfolgen.

Wie bereits erwähnt müssen die Seitenflächen der Blenden absolut planparallel aufeinanderliegen, da sonst ein Spalt entsteht, durch den Leckstrahlen hindurchtreten können, was vermieden werden muß, da derartige Leckstrahlen auf gesundes Gewebe treffen würden. Daß sich ein sehr geringer Spalt auch durch die präziseste Flächenbehandlung nicht völlig über den gesamten Flächenverlauf der aneinandergrenzenden Flächen vermeiden läßt, ist es zweckmäßig, wenn die aneinanderliegenden Seitenflächen derart verlaufen, daß der Spalt nicht parallel zum Strahlengang verläuft. Dazu kann vorgesehen sein, daß die Seitenflächen in Schieberichtung verlaufende komplementär ineinandergreifende Abweichungen bezüglich der Ebenheit der Seitenflächen aufweisen. Es ist bekannt, dafür Stufen oder ähnliches vorzusehen. Deshalb wird auf diese Ausgestaltungen nicht näher eingegangen. Eine gute Lösung zur Vermeidung der erwähnten Leckstrahlen besteht für die erfindungsgemäße Irisblende darin, daß diese zu einer rechtwinklig zur optischen Achse der Strahlen liegenden gedachten Kollimatorebene derart gekippt ist, daß ein Strahl nicht mehr durch einen möglichen Spalt fallen kann. Da ein solcher durch Toleranzen möglicher Spalt bei hoher Präzision der Flächen im Mikrometerbereich liegt, reicht eine entsprechend kleine Kippung um Bogensekunden aus, welche auf die Strahlenformung keine praktische Auswirkung hat.

Der erfindungsgemäße Kollimator kann natürlich außer im Bereich sehr energiereicher Strahlen auch für Röntgengeräte eingesetzt werden, da die Vorteile nicht überlappender Blendenblätter auch dort relevant sind, weil eine geringe Bauhöhe für jedes Gerät von Vorteil ist.

Um eine zusätzliche Abschirmung zu erzielen, kann vorgesehen sein, daß der erfindungsgemäße Kollimator außer der Irisblende eine Festblende aufweist, die sich im Strahlengang befindet und deren Öffnung auf die größtmögliche Öffnung der Irisblende abgestimmt ist.

Die Erfindung wird nachfolgend anhand von in der Zeichnung dargestellten Ausführungsbeispielen erläutert. Es zeigen
- **Fig. 1**: ein einfaches Ausführungsbeispiel einer dreiblättrigen Irisblende zur Erläuterung des Prinzips,
- **Fig. 2**: eine Prinzipskizze des Kollimators,
- **Fig. 3**: eine Führung an den Seitenflächen von Blendenblättern,
- **Fig. 4a, 4b und 4c**: eine Prinzipskizze einer vierblättrigen Irisblende,

- **Fig. 5a, 5b und 5c**: ein Ausführungsbeispiel einer Mechanik zur simultanen Verstellung von Blendenblätter,
- **Fig. 6a und 6b**: eine weitere Ausgestaltungsmöglichkeit einer vierblättrigen Irisblende,
- **Fig. 7a, 7b und 7c**: eine Prinzipskizze einer fünfblättrigen Irisblende,
- **Fig. 8a, 8b und 8c**: eine Prinzipskizze einer sechsblättrigen Irisblende,
- **Fig. 9**: ein weiteres Ausführungsbeispiel einer Mechanik zur simultanen Verstellung von Blendenblättern und
- **Fig. 9a**: eine Einzelheit dieser Mechanik.

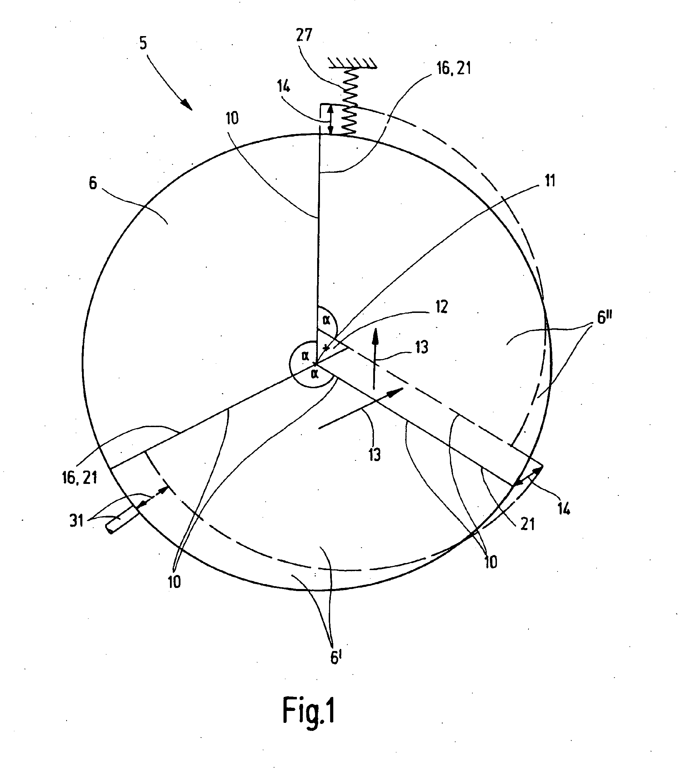

**Fig. 1** zeigt ein einfaches Ausführungsbeispiel einer dreiblättrigen Irisblende 5 zur Erläuterung des Prinzips. Für diese Erläuterung wurde eine dreiblättrige Irisblende 5 deshalb gewählt, da sie aufgrund der wenigen Teile am übersichtlichsten darstellbar ist. Diese Irisblende 5 verfügt über drei Blendenblätter 6, 6' und 6". Bei diesem Ausführungsbeispiel ist das Blendenblatt 6 fest und die Blendenblätter 6' und 6" in Richtung der Pfeile 13 verschiebbar. Im geschlossenen Zustand der Irisblende 5 befinden sich die Winkel α am Mittelpunkt 11, wobei jeder Winkel α durch zwei Seitenflächen 10 der Blendenblätter 6, 6', 6" gebildet ist. Diese Winkel α fallen natürlich bei Irisblenden 5 mit mehr Blendenblättern in entsprechender Weise kleiner aus.

Bei dieser Ausführungsform sind die Blendenblätter 6' und 6" mittels Führungen 21 an den Seitenflächen 10 derart geführt, daß die Seitenflächen 10 fest aufeinanderliegen. Dabei bilden aufgrund der festen Anordnung des Blendenblattes 6 dessen Seitenkanten 10 Linearführungen 16 für die beiden anderen Blendenblätter 6' und 6" und die Führung 21 zwischen den beiden letztgenannten verschiebt sich mit diesen Blendenblättern 6' und 6", wobei diese die Stellwege 14 vollziehen. Dieser Verschiebung dient ein symbolisch dargestellter Antrieb 31 am Blendenblatt 6' und der Rückstellung dient eine Rückstellfeder 27 am Blendenblatt 6". Durch die Schiebebewegung 13 wird eine Blendenöffnung 12 geöffnet, wobei sich die Größe der Blendenöffnung 12 nach dem Maß der zurückgelegten Stellwege 14 richtet.

Günstiger als eine dreieckige Blendenöffnung 12 ist eine viereckige oder eine nahezu runde Form. Dies zeigen später dargestellte und beschriebene Ausführungsbeispiele. Weiterhin ist eine Ausgestaltung zweckmäßiger, bei der sich der Mittelpunkt 11 mit der Öffnung der Irisblende 5 nicht verschiebt, wie dies hier mit den beiden kleinen Kreuzen eingezeichnet ist, sondern bei denen dieser Mittelpunkt 11 erhalten bleibt, wenn sich die Irisblende 5 öffnet. Zu diesem Zweck müssen dann jedoch alle Blendenblätter eine Schiebebewegung 13 vollziehen und es ist daher erforderlich, daß diese Blendenblätter mittels entsprechender Linearführungen gelagert sind. Auch dies wird an den noch folgenden Ausführungsbeispielen erläutert.

**Fig. 2** zeigt eine Prinzipskizze des Kollimators 1 mit einer Irisblende 5. Der Kollimator 1 ist einer Strahlungsquelle 3 zugeordnet, von der Strahlen 2 ausgehen. Der Irisblende 5 vorgeordnet ist eine Festblende 30, die eine Öffnung aufweist, welche der größtmöglichen Öffnung der Irisblende 5 entspricht. Diese Festblende 30 dient dazu, die Strahlen 2 der Strahlungsquelle 3 einzugrenzen und das Auftreten von Streustrahlen so weit wie möglich zu verhindern. Die Irisblende 5 ist hier in einer Schnittdarstellung gezeigt, wobei es sich um eine vierblättrige Irisblende 5 mit Blendenblättern 7, 7', 7", 7'" handelt. Diese wird später noch näher dargestellt. Durch die Öffnung 12 der Irisblende 5 werden die Strahlen 2 auf die Strahlen 2' weiter eingeengt, und zwar derart, daß mit diesen Strahlen 2' beispielsweise die Fläche eines Behandlungsobjekts 4 abgescannt werden kann, wobei bezüglich einer solchen Scannvorrichtung auf die DE 101 57 523 C1 verwiesen wird. Eine solche Scannvorrichtung kann wiederum auf einer Gantry angeordnet sein, so daß es möglich ist, das Behandlungsobjekt 4 von unterschiedlichen Seiten aus zu bestrahlen und so eine maximale Bestrahlung, beispielsweise eines Tumors zu erzielen, bei der gleichzeitig das umliegende Gewebe wesentlich weniger Strahlung erhält.

**Fig. 3** zeigt eine Einzelheit einer Führung 21 zwischen zwei Blendenblättern 32. Die Blendenblätter 32 können beliebige Blendenblätter sein, dergestalt wie sie in dieser Beschreibung vorhanden sind oder natürlich auch einer Irisblende 5, die noch mehr Blendenblätter aufweist. Die hier dargestellte Führung 21 dient dazu, zwei Blendenblätter 32 mit ihren Seitenflächen 10 derart fest zusammenzuhalten, daß möglichst kein Spalt 28 entsteht. Dazu dient auch die in der Führung 21 angeordnete Feder 20, welche an die Blendenblätter 32 angefügte Absätze 38 zusammenpreßt. Solche Führungen 21 können natürlich nur in den Bereichen der Seitenflächen 10 angeordnet sein, die nicht als Teilbereiche 15 für die Bildung einer Blendenöffnung 12 herangezogen werden.

Da sich ein Spalt 28 nie hundertprozentig vermeiden läßt, wird vorgeschlagen, daß die in **Fig. 2** eingezeichnete Blendenebene 29 zu der optischen Achse 33 leicht gekippt wird, und zwar derart, daß keine Strahlen 2 durch einen Spalt 28 zwischen Blendenblättern 32 fallen können. Dies heißt, daß der Winkel β eine leichte Abweichung von 90° hat, wobei in der Regel wenige Bogensekunden ausreichend sind. Ergänzend ist hinzuzufügen, daß der Strahlengang in Fig. 2 wesentlich verkürzt gezeichnet ist. Tatsächlich ist im Verhältnis zur Blendenöffnung 12 der Abstand zur Strahlungsquelle 3 wesentlich größer, so daß die Strahlen 2 im Bereich der Irisblende 5 nur noch eine minimale Abweichung von einem parallelen Verlauf haben, so daß im Gegensatz zur Darstellung der Fig. 2 ein Hindurchtreten von Strahlen 2 durch einen Spalt 28 möglich ist und daher durch die erwähnte Verkippung oder eine andere Maßnahme unterbunden werden sollte. Die Verkippung kann jedoch sehr gering ausfallen, weil aufgrund der hohen Oberflächenqualität und Planheit der Seitenflächen 10 ein Spalt 28 ohnehin nur im Mikrometerbereich auftreten kann.

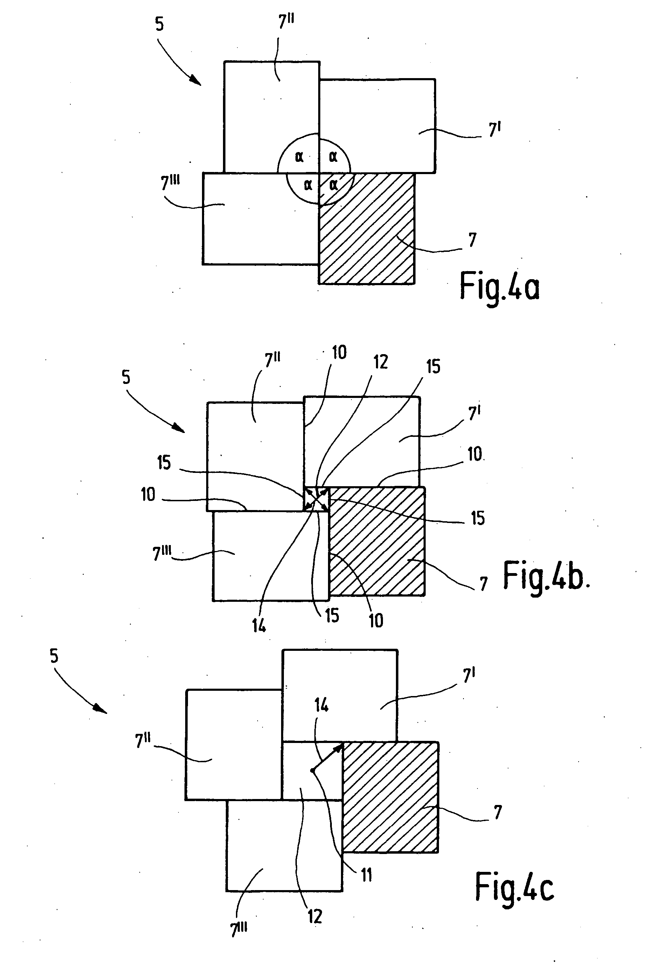

Die **Fig. 4a, 4b und 4c** zeigen eine Prinzipskizze einer vierblättrigen Irisblende 5. Diese verfügt über die Blendenblätter 7, 7', 7"und 7'". In **Fig. 4a** ist die Irisblende 5 geschlossen, wobei die Winkel α, die rechtwinklig sind, aneinanderliegen. In **Fig. 4b** haben sich alle Blendenblätter um denselben Stellweg 14 bewegt, so daß eine Öffnung 12 entstanden ist, welche von Teilbereichen 15 der Seitenflächen 10 der Blendenblätter 7, 7', 7", 7"' gebildet wird. Die Stellwege 14 entsprechen jeweils der Hälfte der beiden Diagonalen der quadratischen Öffnung 12.

**Fig. 4c** zeigt eine weitere Öffnung der Irisblende 5, wobei der Mittelpunkt 11, der in der optischen Achse 33 (siehe Fig. 2) liegt, eingezeichnet ist, und von diesem ausgehend der Stellweg 14, den das linke obere Eck des Blendenblatts 7 vollzogen hat. In entsprechender Weise haben auch die anderen Blendenblätter 7', 7" und 7"' Stellwege 14 vollzogen.

Die **Fig. 5a, 5b und 5c** zeigen ein Ausführungsbeispiel einer Mechanik 22 zur simultanen Verstellung von Blendenblättern. Dargestellt ist dies anhand von vier Blendenblättern 7, 7', 7" und 7"'. Die Mechanik 22 weist ein Stellorgan 25 auf, das über Stellarme 26 verfügt, welche jeweils einem der Blendenblätter 7, 7', 7" und 7'" zugeordnet sind. Die Blendenblätter 7, 7', 7" und 7'" besitzen Führungsbolzen 34 und zwar jeweils zwei, welche in Linearführungen 16 gelagert sind. Wenn sich das Stellorgan 25 in Richtung des Pfeils 35 dreht, schieben die Stellarme 26 die Bolzen 34 entlang der Linearführungen 16 und bewirken somit die oben beschriebenen Stellwege 14 der Blendenblätter 7, 7', 7", 7"'.

**Fig. 5b** zeigt bereits eine Öffnung 12, die in **Fig. 5c** noch weiter geöffnet ist. In **Fig. 5c** sind auch die Schiebebewegungen 13 eingezeichnet, sowie die mögliche Anordnung von Rückstellfedern 27, welche die Irisblende 5 wieder schließen, wenn das Stellorgan 25 entgegen der Richtung des Pfeils 35 zurückbewegt wird.

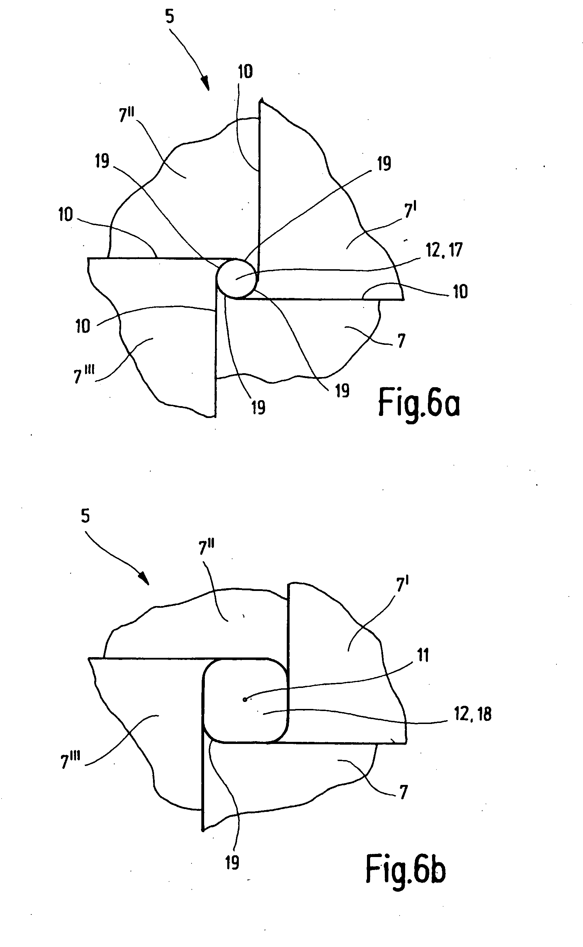

Die **Fig. 6a und 6b** zeigen eine weitere Ausgestaltungsmöglichkeit einer vierblättrigen Irisblende 5. Dabei weisen die Blendenblätter 7, 7', 7" und 7"' an den vorderen Enden ihrer Seitenflächen 10 nasenartig auskragende Kreisbögen 19 auf. Bei dieser Ausgestaltungsmöglichkeit kann die Irisblende 5 nicht völlig geschlossen werden, da eine Stellbewegung 13 hier nur soweit möglich ist, bis die Kreisbögen 19, die jeweils ein Vierteilkreis sind, sich zu einer runden Öffnung 17 zusammenfügen. Dies ist dann die kleinstmögliche Öffnung 12. Wird diese Irisblende 5 in entsprechender Weise wie oben beschrieben geöffnet, so entsteht eine quadratische Öffnung 18, bei der die Kreisbögen 19 gerundete Ecken bilden. Dies ist in **Fig. 6b** gezeigt. Auf die Vorteile einer derartigen Ausgestaltung wurde oben bereits verwiesen.

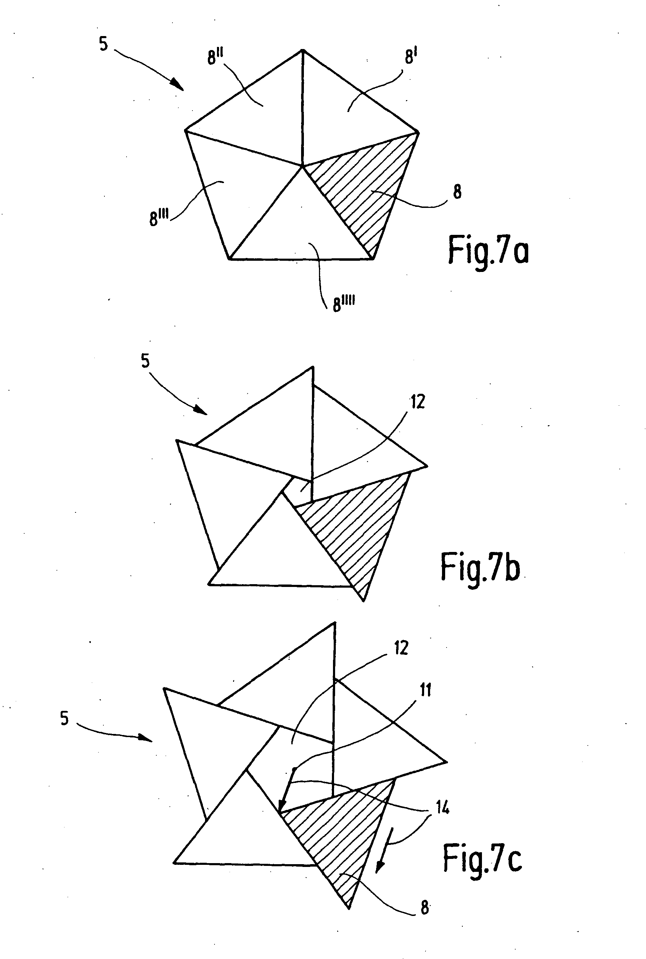

Die **Fig. 7a, 7b und 7c** zeigen eine Prinzipskizze einer fünfblättrigen Irisblende 5. Auch hier vollziehen alle Blendenblätter 8, 8', 8", 8'"' und 8"" simultane Stellbewegungen, um eine Öffnung 12 zu bilden, wie sie in den **Fig. 7b und 7c** dargestellt ist. In der **Fig. 7c** ist noch verdeutlicht, welchen Stellweg 14 beispielsweise das schraffiert hervorgehobene Blendenblatt 8 vollzieht, wobei der vom Mittelpunkt 11 ausgehende Stellweg 14 den Weg beschreibt, den die Ecke des Blendenblatts 8 vollzogen hat, die jetzt an der Pfeilspitze liegt.

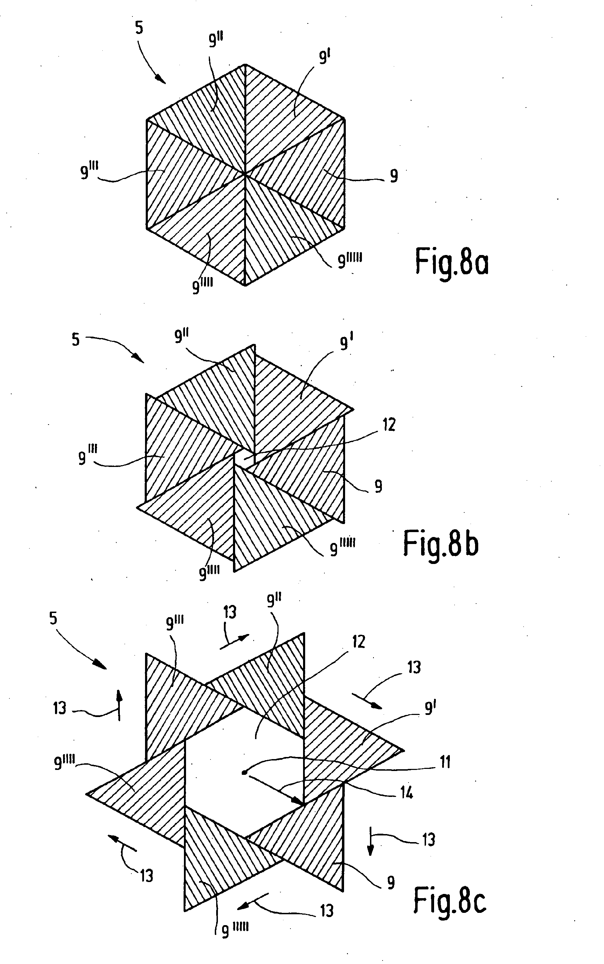

**Fig. 8a, 8b und 8c** zeigen eine Prinzipskizze einer sechsblättrigen Irisblende 5. Die Darstellungen entsprechen den zuvor erläuterten Darstellungen, wobei die Blendenblätter 9, 9', 9", 9"', 9"" und 9""' in verschiedenen Schraffuren gezeichnet sind, damit die Positionen dieser Blendenblätter bei den mit den **Fig. 8b und 8c** gezeigten Öffnungsbewegungen besser erkennbar sind. In **Fig. 8c** ist noch mit den Pfeilen 13 die Schiebebewegung eines jeden der Blendenblätter 9, 9', 9", 9'", 9"" und 9""' eingezeichnet. Die Stellbewegung 14 ist mit einem vom Mittelpunkt 11 ausgehenden Pfeil für das Eck an der Pfeilspitze dargestellt, das zum Blendenblatt 9' gehört.

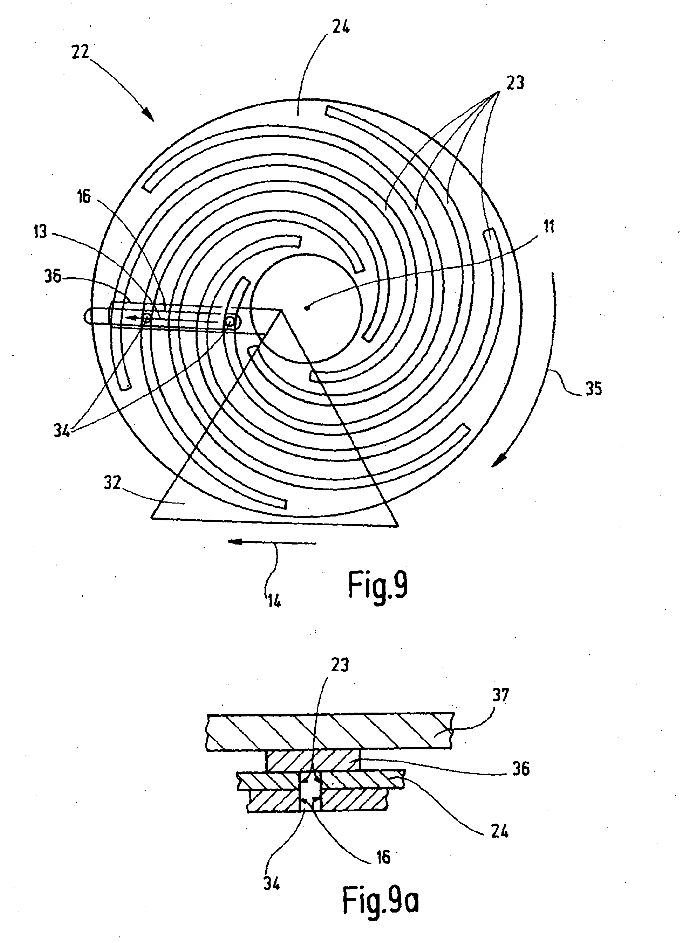

Die **Fig. 9** zeigt ein weiteres Ausführungsbeispiel einer Mechanik 22 zur simultanen Verstellung von Blendenblättern, wobei die **Fig. 9a** eine Einzelheit dieser Mechanik 22 als Schnitt quer zu einem Haltearm 36 zeigt. Es handelt sich bei dem Ausführungsbeispiel um eine Kurvenscheibe 24, welche Stellkurven 23 aufweist. Die Blendenblätter 32 - es kann sich um beliebig ausgestaltete handeln - sind hier jeweils mit einem Haltearm 36 ausgestattet, welcher jeweils zwei Führungsbolzen 34 trägt. Dabei wurde die Darstellung auf ein Blendenblatt 32 beschränkt. Die Führungsbolzen 34 erstrecken sich durch die als Nuten ausgebildeten Stellkurven 23 der Kurvenscheibe 24 hindurch und laufen zusätzlich in Linearführungen 16. Auf diese Weise wird mittels einer Drehung in Richtung des Pfeils 35 der Kurvenscheibe 24 erreicht, daß das Blendenblatt 32 eine Schiebebewegung in Richtung des Pfeils 13 vollzieht. Bewegt sich dagegen die Kurvenscheibe 24 entgegen des Pfeils 35, so wird das Blendenblatt 32 wieder entgegen dem Stellweg 14 zurückgestellt. Diese Stellwege 14 vollziehen dann alle angeordneten Blendenblätter 32 immer in simultaner Weise.

Es wurde hier nicht festgelegt, wie viele Blendenblätter vorhanden sind, da derartige Kurvenscheiben 24 für eine nahezu beliebige Anzahl von Blendenblättern 32 eingesetzt werden können. Entsprechend der Anzahl der Blendenblätter 32 und entsprechend der gewünschten Öffnung 12 und damit der gewünschten Stellwege 14 richtet sich die Anzahl und der Verlauf der Stellkurven 23. Dabei zeigt die Fig. 9a noch eine Deckplatte 37, die die Mechanik 22 zweckmäßigerweise abdeckt. Statt der Haltearme 36 kann natürlich auch eine entsprechende Anordnung der Führungsbolzen 34 direkt am jeweiligen Blendenblatt 32 vorgenommen werden.

Die dargestellten Ausführungsbeispiele geben lediglich einen kleinen Ausschnitt von Möglichkeiten wieder. Insbesondere Lagerung und Stellmechanik kann auch auf andere Weise ausgebildet sein, wie dies eingangs bereits erwähnt wurde. Insbesondere ist es auch möglich, die Anzahl der Blendenblätter noch zu vergrößern, um eine möglichst runde Ausgestaltung der Blendenöffnung 12 zu erzielen. Auch könnten z. B. anstatt der Führungen 21 an den Seitenflächen 10 Schwalbenschwanzführungen vorgesehen sein, wenn diese nur in den Bereichen angeordnet sind, die nicht zur Bildung der Öffnung 12 dienen. Auch die Federn 20 könnten an den Außenseiten der Blendenblätter 6, 6', 6" oder 7, 7', 7", 7'" oder 8, 8', 8", 8"', 8"" oder 9, 9', 9", 9"', 9" ", 9'"" angeordnet sein, um sie jeweils gegen die Seitenflächen 10 der beiden angrenzenden Blendenblätter zu drücken. Eine weitere Möglichkeit, einen guten gegenseitigen Halt der Seitenflächen 10 zu erzielen, wäre eine elastische Umfassung aller Blendenblätter einer Irisblende 5. Zahlreiche weitere Ausgestaltungsmöglichkeiten sind denkbar.

### Bezugszeichenliste

- 1: Kollimator
- 2: Strahlen
- 2': Strahlen durch den Kollimator begrenzt
- 3: Strahlungsquelle
- 4: Behandlungsobjekt (Diagnose- oder Strahlentherapie)
- 5: Irisblende
- 6, 6', 6": Blendenblätter bei einer dreiblättrigen Irisblende
- 7, 7', 7", 7"': Blendenblätter bei einer vierblättrigen Irisblende
- 8, 8', 8", 8"', 8"": Blendenblätter bei einer fünfblättrigen Irisblende
- 9, 9', 9", 9"', 9"": Blendenblätter bei einer sechsblättrigen Irisblende
- 10: Seitenflächen
- 11: Mittelpunkt
- 12: Öffnung / Blendenöffnung
- 13: Pfeile: Schiebebewegung
- 14: Stellwege
- 15: Teilbereiche der Seitenflächen, die die Öffnung bilden
- 16: Linearführungen
- 17: runde Öffnung
- 18: quadratische Öffnung mit gerundeten Ecken
- 19: Kreisbögen (nasenartig auskragend)

- 20: Federn
- 21: Führungen an den Seitenflächen
- 22: Mechanik
- 23: Stellkurven
- 24: Kurvenscheibe
- 25: Stellorgan
- 26: Stellarme
- 27: Rückstellfedern
- 28: Spalt (durch Toleranzen bedingt)
- 29: Blendenebene
- 30: Festblende
- 31: Antrieb (symbolisch)
- 32: beliebige Blendenblätter
- 33: optische Achse
- 34: Führungsbolzen an den Blendenblättern
- 35: Pfeil: Drehrichtung Kurvenscheibe oder Stellorgan
- 36: Haltearm eines Blendenblattes
- 37: Deckplatte
- 38: Absätze

- α: Winkel zwischen den Seitenflächen der Blendenblätter
- β: Winkel zwischen optischer Achse und Blendenebene

## Patentansprüche

1. Kollimator (1) und Scannvorrichtung, wobei der Kollimator (1) zum Begrenzen eines Bündels energiereicher Strahlen (2) dient, das von einer im wesentlichen punktförmigen Strahlungsquelle (3) ausgehend auf ein Behandlungsobjekt (4) gerichtet ist und insbesondere der stereotaktischen Konformationsbestrahlung von Tumoren dient, wobei der Kollimator (1) eine Blende aufweist und die Scannvorrichtung die Fläche eines Behandlungsobjekts (4) aus einer Raumwinkelausrichtung mittels der durch die Blende begrenzten Strahlen (2') abscannt,
**dadurch gekennzeichnet,**
**daß** der Kollimator (1) als strahlbegrenzendes Mittel eine Irisblende (5) mit mindestens drei Blendenblätter (6, 6', 6" oder 7, 7', 7", 7"' oder 8, 8', 8", 8"', 8"" oder 9, 9', 9", 9"', 9"", 9""') aufweist, die gleiche Winkel (α) einschließende, aneinanderliegende Seitenflächen (10) aufweisen, wobei die Blendenblätter (6, 6', 6" oder 7, 7', 7", 7"' oder 8, 8', 8", 8"', 8"" oder 9, 9', 9", 9"', 9"", 9""') eine strahlbegrenzende Öffnung (12) **dadurch** freigeben, daß mittels eines Antriebs (31) eine Schiebebewegung (13) entlang der Seitenflächen (10) durch eine höchstens um eines verminderte Anzahl von Blendenblättern (6, 6', 6" oder 7, 7', 7", 7"' oder 8, 8', 8", 8"', 8"" oder 9, 9', 9", 9"', 9"", 9""') stattfindet.

2. Kollimator und Scannvorrichtung nach Anspruch 1,
**dadurch gekennzeichnet,**
**daß** als Scannvorrichtung ein Roboterarm dient, wobei sich Strahlenquelle (3) und Irisblende (5) an dem Roboterarm befinden, der um das Behandlungsobjekt (4) bewegbar ist, um ein räumliches Gebilde aus verschiedenen Raumwinkelausrichtungen abzuscannen.

3. Kollimator und Scannvorrichtung nach Anspruch 1,
**dadurch gekennzeichnet,**
**daß** Strahlenquelle (3), Kollimator (1) und Scannvorrichtung mittels einer Gantry in verschiedene Raumwinkelausrichtungen der durch den Kollimator (1) begrenzten Strahlen (2') zum Behandlungsobjekt (4) bringbar sind.

4. Kollimator und Scannvorrichtung nach einem der Ansprüche 1 bis 3,
**dadurch gekennzeichnet,**
**daß** seine Abschirmungsfähigkeit für energiereiche Strahlen einer Strahlungsquelle (3) im Megavoltbereich ausgelegt ist.

5. Kollimator und Scannvorrichtung nach Anspruch 4,
**dadurch gekennzeichnet,**
**daß** die Dicke der Blendenblätter (6, 6', 6" oder 7, 7', 7", 7"' oder 8, 8', 8", 8"', 8"" oder 9, 9', 9", 9"', 9"", 9""') zwischen 6 und 10 cm liegt.

6. Kollimator und Scannvorrichtung nach einem der Ansprüche 1 bis 5,
**dadurch gekennzeichnet,**
**daß** eine Schiebebewegung (13) aller Blendenblätter (6, 6', 6" oder 7, 7', 7", 7'' oder 8, 8', 8", 8"', 8"" oder 9, 9', 9", 9''', 9"", 9""') um gleiche Stellwege (14) erfolgt, so daß nach der Stellbewegung die Öffnung (12) durch Teilbereiche (15) der Seitenflächen (10) gebildet wird, die gleiche Entfernungen vom Mittelpunkt (11) aufweisen.

7. Kollimator und Scannvorrichtung nach einem der Ansprüche 1 bis 6,
**dadurch gekennzeichnet,**
**daß** die Blendenblätter (6, 6', 6" oder 7, 7', 7", 7"' oder 8, 8', 8", 8"', 8"" oder 9, 9', 9", 9"', 9"", 9""') mittels in Richtung der Schiebebewegung (13) verlaufender Linearführungen (16) gelagert sind.

8. Kollimator und Scannvorrichtung nach einem der Ansprüche 1 bis 7,
**dadurch gekennzeichnet,**
**daß** die Irisblende (5) vier Blendenblätter (7, 7', 7", 7"') aufweist.

9. Kollimator und Scannvorrichtung nach Anspruch 8,
**dadurch gekennzeichnet,**
**daß** die jeweilige, die Öffnung (12) bildende Seitenfläche (10) an ihrem inneren Ende in einen nasenartig auskragenden einen Viertelkreis bildenden Kreisbogen (19) übergeht, so daß die vier Blendenblätter (7, 7', 7", 7"') wahlweise eine runde Öffnung (17) oder quadratische Öffnungen mit gerundeten Ecken (18) verschiedenen Größen bilden können.

10. Kollimator und Scannvorrichtung nach einem der Ansprüche 1 bis 7,
**dadurch gekennzeichnet,**
**daß** die Irisblende (5) mindestens sechs Blendenblätter (9, 9', 9", 9"', 9"", 9""') aufweist.

11. Kollimator und Scannvorrichtung nach einem der Ansprüche 1 bis 10,
**dadurch gekennzeichnet,**
**daß** Kraftbeaufschlagungen vorgesehen sind, die die Seitenflächen (10) der Blendenblätter (6, 6', 6" oder 7, 7', 7", 7"' oder 8, 8', 8", 8"', 8"" oder 9, 9', 9", 9"', 9"", 9""') gegeneinanderdrücken.

12. Kollimator und Scannvorrichtung nach Anspruch 11,
**dadurch gekennzeichnet,**
**daß** auf die Blendenblätter (6, 6', 6" oder 7, 7', 7", 7"' oder 8, 8', 8", 8"', 8"" oder 9, 9', 9", 9"', 9"", 9""') Federn (20) wirken, die die Kraftbeaufschlagungen bewirken.

13. Kollimator und Scannvorrichtung nach Anspruch 11 oder 12,
**dadurch gekennzeichnet,**
**daß** die Seitenflächen (10) in ihren nicht zur Bildung einer Öffnung (12) dienenden aneinanderliegenden Bereichen gemeinsame Führungen (21) aufweisen, in denen die Seitenflächen (10) der aneinandergrenzenden Blendenblätter (6, 6', 6" oder 7, 7', 7", 7"' oder 8, 8', 8", 8"', 8"" oder 9, 9', 9", 9"', 9"", 9""') gegenseitig verschiebbar sind.

14. Kollimator und Scannvorrichtung nach einem der Ansprüche 1 bis 13,
**dadurch gekennzeichnet,**
**daß** die Schiebebewegung (13) der Blendenblätter (6, 6', 6" oder 7, 7', 7", 7"' oder 8, 8', 8", 8"', 8"" oder 9, 9', 9", 9"', 9"", 9""') **dadurch** erfolgt, daß mindestens ein Blendenblatt (6, 6' oder 6"; 7, 7', 7" oder 7"'; 8, 8', 8", 8"' oder 8""; 9, 9', 9", 9"', 9"" oder 9""') angetrieben wird.

15. Kollimator und Scannvorrichtung nach Anspruch 14,
**dadurch gekennzeichnet,**
**daß** alle Blendenblätter (6, 6', 6" oder 7, 7', 7", 7"' oder 8, 8', 8", 8"', 8"" oder 9, 9', 9", 9"', 9"", 9""') simultan angetrieben werden.

16. Kollimator und Scannvorrichtung nach Anspruch 15,
**dadurch gekennzeichnet,**
**daß** für jedes Blendenblatt (6, 6', 6" oder 7, 7', 7", 7"' oder 8, 8', 8", 8"', 8"" oder 9, 9', 9", 9"', 9"", 9""') ein Antrieb vorgesehen ist, wobei durch eine elektronische Steuerung eine simultane Bewegung erfolgt.

17. Kollimator und Scannvorrichtung nach Anspruch 15,
**dadurch gekennzeichnet,**
**daß** ein Antrieb über eine Mechanik (22) alle Blendenblätter (6, 6', 6" oder 7, 7', 7", 7"' oder 8, 8', 8", 8"', 8"" oder 9, 9', 9", 9'", 9"", 9""') simultan antreibt.

18. Kollimator und Scannvorrichtung nach Anspruch 17,
**dadurch gekennzeichnet,**
**daß** die Mechanik (22) die Blendenblätter (6, 6', 6" oder 7, 7', 7", 7'" oder 8, 8', 8", 8"', 8"" oder 9, 9', 9", 9"', 9"", 9""') über, auf einer um den Mittelpunkt (11) drehbaren Kurvenscheibe (24) spiralförmig angeordnete Stellkurven (23) antreibt.

19. Kollimator und Scannvorrichtung nach Anspruch 17,
**dadurch gekennzeichnet,**
**daß** die Mechanik (22) ein um den Mittelpunkt (11) drehbares Stellorgan (25) aufweist, das auf jedes Blendenblatt (6, 6', 6" oder 7, 7', 7", 7"' oder 8, 8', 8", 8"', 8"" oder 9, 9', 9", 9"', 9"", 9""') mit einem Stellarm (26) wirkt.

20. Kollimator und Scannvorrichtung nach Anspruch 19,
**dadurch gekennzeichnet,**
**daß** der Schiebebewegung (13) mittels der Stellarme (26) Rückstellfedern (27) entgegenwirken.

21. Kollimator und Scannvorrichtung nach einem der Ansprüche 1 bis 20,
**dadurch gekennzeichnet,**
**daß** die aneinanderliegenden Seitenflächen (10) der Blenden (6, 6', 6" oder 7, 7', 7", 7"' oder 8, 8', 8", 8"', 8"" oder 9, 9', 9", 9"', 9"", 9""') derart verlaufen, daß ein durch Toleranzen bedingter Spalt (28) nicht parallel zum Strahlengang verläuft.

22. Kollimator und Scannvorrichtung nach Anspruch 21,
**dadurch gekennzeichnet,**
**daß** die Seitenflächen (10) in Schieberichtung verlaufende komplementär ineinandergreifende Abweichungen bezüglich der Ebenheit der Seitenflächen (10) aufweisen.

23. Kollimator und Scannvorrichtung nach Anspruch 21,
**dadurch gekennzeichnet,**
**daß** die Irisblende (5) zu einer rechtwinklig zur optischen Achse liegenden gedachten Blendenebene (29) derart gekippt ist, daß ein Strahl (2) nicht mehr durch einen Spalt (28) fallen kann.

24. Kollimator und Scannvorrichtung nach einem der Ansprüche 1 bis 23,
**dadurch gekennzeichnet,**
**daß** sich außer der Irisblende (5) eine Festblende (30) für eine zusätzliche Abschirmung im Strahlengang befindet, deren Öffnung auf die größtmögliche Öffnung (12) der Irisblende (5) abgestimmt ist.

## Claims

1. A collimator (1) and scanning device, which collimator (1) serves to delimit a bundle of high-energy radiation (2) emitted by a substantially punctiform radiation source (3) and directed onto a therapy object (4) and serves, in particular, to effect stereotactic conformation irradiation of tumors, and said collimator (1) comprises a diaphragm and said scanning device scans the surface of a therapy object (4) within a solid angle with the radiation (2') delimited by said diaphragm
**characterized in that**
said collimator (1) has as beam delimiting means an iris diaphragm (5) comprising at least three iris blades (6, 6', 6", or 7, 7', 7", 7"', or 8, 8', 8", 8"', 8"", or 9, 9', 9", 9"', 9"", 9""') which have juxtaposed side faces (10) enclosing in each case the same angle (α), which iris blades (6, 6', 6", or 7, 7', 7", 7"', or 8, 8', 8", 8"', 8"", or 9, 9', 9", 9"', 9"", 9""') form a beam delimiting aperture (12) **in that** driving means (31) causes displacement (13), along their side faces (10), of the total number of iris leaves (6, 6', 6", or 7, 7', 7", 7"', or 8, 8', 8", 8"', 8"", or 9, 9', 9", 9"', 9"", 9""') less at most one.

2. The collimator and scanning device as defined in claim 1,
**characterized in that**
a robot arm serves as scanning device, and said source of radiation (3) and said iris diaphragm (5) are disposed on said robot arm, which is capable of moving over said therapy object (4) in order to scan a spatial entity from various solid angle orientations.

3. The collimator and scanning device as defined in claim 1,
**characterized in that**
said source of radiation (3), said collimator (1), and said scanning device are capable of being moved by means of a gantry crane into various solid angle orientations of the radiation (2') delimited by said collimator (1) relative to said therapy object (4).

4. The collimator and scanning device as defined in any one of claims 1 to 3,
**characterized in that**
its screening capacity is suitable for high-energy radiation from a radiation source (3) in the megavolt range.

5. The collimator and scanning device as defined in claim 4,
**characterized in that**
the thickness of the iris blades (6, 6', 6", or 7, 7', 7", 7"', or 8, 8', 8", 8"', 8"", or 9, 9', 9", 9"', 9"", 9""') is from 6 cm to 10 cm.

6. The collimator and scanning device as defined in any one of claims 1 to 5,
**characterized in that**
the displacement (13) of all of said iris blades (6, 6', 6", or 7, 7', 7", 7"', or 8, 8', 8", 8"', 8"", or 9, 9', 9", 9"', 9"", 9""') occurs through identical distances (14) such that after such a setting movement the aperture (12) is formed by subregions (15) of the side faces (10) positioned at the same distance from the center (11).

7. The collimator and scanning device as defined in any one of claims 1 to 6,
**characterized in that**
said iris blades (6, 6', 6", or 7, 7', 7", 7"', or 8, 8', 8", 8"', 8"", or 9, 9', 9", 9"', 9"", 9""') are mounted by means of linear guides (16) extending in the direction of displacement (13).

8. The collimator and scanning device as defined in any one of claims 1 to 7,
**characterized in that**
said iris diaphragm (5) has four iris blades (7, 7', 7", 7"').

9. The collimator and scanning device as defined in claim 8,
**characterized in that**
each respective side face (10) forming the aperture (12) merges at its inner end into a nose-like protrusion (19) in the form of an arc of a circle forming a quadrant, such that said four iris blades (7, 7', 7", 7"') can optionally form a round aperture (17) or various sizes of square apertures with rounded corners (18).

10. The collimator and scanning device as defined in any one of claims 1 to 7,
**characterized in that**
said iris diaphragm (5) has at least six iris blades (9, 9', 9", 9"', 9"", 9').

11. The collimator and scanning device as defined in any one of claims 1 to 10,
**characterized in that**
force application means are provided which press the side faces (10) of the iris blades (6 , 6' , 6" or 7 7', 7", 7", or 8 8', 8", 8"', 8"", or 9, 9', 9", 9'", 9"", 9""') together.

12. The collimator and scanning device as defined in claim 11,
**characterized in that**
springs (20) act on said iris blades (6, 6', 6", or 7, 7', 7", 7"', or 8, 8', 8", 8"', 8"" or 9, 9', 9", 9"', 9"", 9""') to form said force application means.

13. The collimator and scanning device as defined in claim 11 or claim 12
**characterized in that**
said side faces (10) have, in their juxtaposed regions not serving to form an aperture (12), common guides (21) in which the side faces (10) of the juxtaposed iris blades (6, 6', 6", or 7, 7', 7", 7"', or 8, 8', 8", 8"', 8"", or 9, 9', 9", 9"', 9"", 9""') are displaceable relatively to each other.

14. The collimator and scanning device as defined in any one of claims 1 to 13,
**characterized in that**
the displacement (13) of said iris blades (6, 6', 6", or 7, 7', 7", 7"', or 8, 8', 8", 8"', 8"", or 9, 9', 9", 9"', 9"", 9""') is carried out by moving at least one iris blade (6, 6', or 6"; 7, 7', 7", or 7"'; 8, 8', 8", 8"', or 8""; 9, 9', 9", 9"', 9"", or 9""').

15. The collimator and scanning device as defined in claim 14,
**characterized in that**
all of the iris blades (6, 6', 6", or 7, 7', 7", 7"', or 8, 8', 8", 8"', 8"", or 9, 9', 9", 9"', 9"", 9""') are driven concurrently.

16. The collimator and scanning device as defined in claim 15,
**characterized in that**
a drive is provided for each of the iris blades (6, 6', 6", or 7, 7', 7", 7"', or 8, 8', 8", 8"', 8"", or 9, 9', 9", 9"', 9"", 9""'), concurrent motion thereof being effected by electronic control means.

17. The collimator and scanning device as defined in claim 15,
**characterized in that**
one drive concurrently drives all of the iris blades (66', 6", or 7, 7', T'7"', or 8, 8', 8", 8"', 8"", or 9, 9', 9", 9"', 9"", 9""') via mechanical means (22).

18. The collimator and scanning device as defined in claim 17,
**characterized in that**
said mechanical means (22) drives the iris blades (6, 6', 6", or 7, 7', 7" 7"', or 8, 8', 8", 8"', 8"", or 9, 9', 9", 9"', 9"", 9""') via spirally disposed cams (23) mounted for rotation about the center (11) of a rotatable cam wheel (24).

19. The collimator and scanning device as defined in claim 17,
**characterized in that**
said mechanical means (22) comprises a regulating unit (25) mounted for rotation about said center (11) and adapted to act on each iris blade (6, 6', 6", or 7, 7', 7", 7"', or 8, 8', 8", 8"', 8"", or 9, 9', 9", 9"', 9"", 9""') by way of a control arm (26).

20. The collimator and scanning device as defined in claim 19,
**characterized in that**
displacement (13) caused by said control arms (26) is counteracted by return springs (27).

21. The collimator and scanning device as defined in any one of claims 1 to 20,
**characterized in that**
said juxtaposed side faces (10) of said diaphragms (6, 6', 6", or 7, 7', 7", 7"', or 8, 8', 8", 8"', 8"", or 9, 9', 9", 9"', 9"", 9""') are shaped in such a manner that a gap (28) resulting from tolerances does not extend parallel to the optical path.

22. The collimator and scanning device as defined in claim 21,
**characterized in that**
said side faces (10) show complementary deviations in terms of the planarity of said side faces (10), which deviations interengage and extend in the sliding direction.

23. The collimator and scanning device as defined in claim 21,
**characterized in that**
said iris diaphragm (5) is inclined toward an imaginary diaphragm plane (29) that is orthogonal to the optical axis in such a manner that a beam (2) can no longer pass through a gap (28).

24. The collimator and scanning device as defined in any one of claims 1 to 23,
**characterized in that**
in addition to said iris diaphragm (5) a fixed diaphragm (30) for additional screening is present in the optical path and has an aperture matching the maximum possible aperture (12) of said iris diaphragm (5).

## Revendications

1. Collimateur (1) et scanner, le collimateur (1) servant à délimiter un faisceau de rayon à grande énergie (2) qui est dirigé depuis une source de rayonnement (3) sensiblement ponctuelle sur un objet de traitement (4) et en particulier au rayonnement de conformation stéréotactique de tumeurs, le collimateur (1) présentant un diaphragme et le scanner balayant la surface d'un objet de traitement (4) depuis une orientation angulaire spatiale au moyen de rayons (2') limités par l'obturateur,
**caractérisés en ce que** le collimateur (1) présente en tant que moyen de délimitation de rayonnement un diaphragme iris (5) avec au moins trois lamelles de diaphragme(6, 6', 6 " ou 7, 7', 7'', 7''' ou 8, 8', 8'', 8"', 8"" ou 9, 9', 9", 9"', 9" " , 9" " ') qui présentent des surfaces latérales juxtaposées formant un angle (α), et **en ce que** les lamelles de diaphragme (6, 6', 6" ou 7, 7', 7", 7"' ou 8, 8', 8", 8"', 8"" ou 9, 9', 9", 9"', 9"", 9""') libèrent une ouverture (12) délimitant le rayonnement, de façon qu'il se produit un déplacement coulissant (13) le long des surfaces latérales (10) au moyen d'un entraînement (31), tout au plus, d'un nombre réduit de lamelles de diaphragme (6, 6', 6 " ou 7, 7', 7", 7"' ou 8, 8', 8'', 8"', 8"" ou 9, 9', 9", 9"', 9"", 9""',

2. Collimateur et scanner selon la revendication 1, **caractérisés en ce qu'**en tant que scanner, on utilise un bras de robot, la source de rayonnement (3) et le diaphragme iris (5) se trouvant sur le bras de robot qui est mobile autour de l'objet de traitement (4) pour balayer une formation spatiale depuis différentes orientations angulaires spatiales.

3. Collimateur et scanner selon la revendication 1, **caractérisés en ce que** la source de rayonnement (3), le collimateur (1) et le scanner peuvent être amenés au moyen d'un portique dans différentes orientations spatiales angulaires des rayons (2') limités par le collimateur (1) vers l'objet de traitement (4).

4. Collimateur et scanner selon l'une des revendications 1 à 3, **caractérisé en ce que** sa capacité d'écran pour les rayons hautement énergétiques d'une source de rayonnement (3) est placée dans la plage des mégavolts.

5. Collimateur et scanner selon la revendication 4, **caractérisé en ce que** l'épaisseur des lamelles de diaphragme (6, 6', 6" ou 7, 7', 7", 7'"' ou 8, 8', 8", 8"', 8"" ou 9, 9', 9", 9"', 9"", 9""') est comprise entre 6 et 10 cm.

6. Collimateur et scanner selon l'une des revendications 1 à 5, **caractérisé en ce qu'**un déplacement latéral (13) de toutes les lamelles de diaphragme (6, 6', 6" ou 7, 7', 7", 7"' ou 8, 8', 8", 8"', 8"" ou 9, 9', 9", 9"', 9"", 9""') s'effectue sur les mêmes parcours de réglage (14) de sorte qu'après le mouvement de réglage, l'ouverture (12) est formée par des zones partielles (15) des surfaces latérales (10) qui présentent les mêmes éloignements avec le point central (11).

7. Collimateur et scanner selon l'une des revendications 1 à 6, **caractérisés en ce que** les lamelles de diaphragme (6, 6', 6" ou 7, 7', 7", 7'" ou 8, 8', 8", 8"', 8"" ou 9, 9', 9", 9"', 9"", 9""') sont logées au moyen de guides linéaires (16) s'étendant dans la direction du déplacement coulissant (13).

8. Collimateur et scanner selon l'une des revendications 1 à 7, **caractérisés en ce que** le diaphragme iris (5) présente quatre lamelles de diaphragme (7, 7', 7", 7"').

9. Collimateur et scanner selon la revendication 8, **caractérisés en ce que** la surface latérale (10) respective formant l'ouverture (12) passe à son extrémité intérieure en un arc de cercle (19) formant un quart de cercle faisant saillie en ergot, de sorte que les quatre lamelles de diaphragme (7, 7', 7", 7"') peuvent former au choix une ouverture ronde (17) ou une ouverture carrée avec des coins arrondis (18) de grandeurs différentes.

10. Collimateur et scanner selon l'une des revendications 1 à 7, **caractérisés en ce que** le diaphragme iris (5) présente au moins six lamelles de diaphragme (9, 9', 9", 9"', 9"", 9""').

11. Collimateur et scanner selon l'une des revendications 1 à 10, **caractérisés en ce qu'**il est prévu des applications de force qui pressent les surfaces latérales des lamelles de diaphragme (6, 6', 6" ou 7, 7', 7", 7"' ou 8, 8', 8", 8"', 8"" ou 9, 9', 9", 9"', 9"", 9""') les unes contre les autres.

12. Collimateur et scanner selon la revendication 11, **caractérisé en ce que** des ressorts (20), qui provoquent l'application de forces, agissent sur les lamelles de diaphragme (6, 6', 6" ou 7, 7', 7", 7'" ou 8, 8', 8", 8"', 8"" ou 9, 9', 9", 9"', 9"", 9""')

13. Collimateur et scanner selon la revendication 11 ou 12, **caractérisé en ce que** les surfaces latérales (10) présentent dans leurs zones adjacentes ne servant pas à la formation d'une ouverture (12), des guides communs (21) dans lesquels les surfaces latérales (10) des lamelles de diaphragme (6, 6', 6" ou 7, 7', 7", 7'" ou 8, 8', 8", 8"', 8"" ou 9, 9', 9", 9"', 9"", 9""') sont coulissantes les unes par rapport aux autres.

14. Collimateur et scanner selon l'une des revendications 1 à 33, **caractérisé en ce que** le déplacement coulissant (13) des lamelles de diaphragme (6, 6', 6" ou 7, 7', 7", 7"' ou 8, 8', 8", 8"', 8"" ou 9, 9', 9", 9"', 9"", 9""') s'effectue par le fait qu'au moins une lamelle de diaphragme (6, 6', 6" ou 7, 7', 7", 7'" ou 8, 8', 8", 8"', 8"" ou 9, 9', 9", 9"', 9"", 9""') est entraînée.

15. Collimateur et scanner selon la revendication 14, **caractérisé en ce que** toutes les lamelles de diaphragme (6, 6', 6" ou 7, 7', 7", 7"' ou 8, 8', 8", 8"', 8"" ou 9, 9', 9", 9"' 9"" 9""') sont entraînées simultanément.

16. Collimateur et scanner selon la revendication 15, **caractérisé en ce qu'**il est prévu un entraînement pour chaque lamelle de diaphragme (6, 6', 6" ou 7, 7', 7", 7"' ou 8, 8', 8", 8"', 8"" ou 9, 9', 9", 9"', 9"", 9""'), un déplacement simultané s'effectuant par une commande électronique.

17. Collimateur et scanner selon la revendication 15, **caractérisé en ce qu'**un entraînement entraîne simultanément par une mécanique (22) toutes les lamelles de diaphragme (6, 6', 6" ou 7, 7', 7", 7"' ou 8, 8', 8", 8"', 8"" ou 9, 9', 9", 9"', 9"", 9""').

18. Collimateur et scanner selon la revendication 17, **caractérisé en ce que** la mécanique (22) entraîne les lamelles de diaphragme (6, 6', 6" ou 7, 7', 7", 7"' ou 8, 8', 8", 8"', 8"" ou 9, 9', 9", 9"', 9"", 9""') par des cames de réglages (23) disposées en forme de spirale sur un disque à came (24).

19. Collimateur et scanner selon la revendication 17, **caractérisé en ce que** la mécanique (22) présente un organe de réglage (25) rotatif autour du point central (11), qui agit sur chaque lamelle de diaphragme (6, 6', 6" ou 7, 7', 7", 7'" ou 8, 8', 8", 8"', 8"" ou 9, 9', 9", 9"', 9"", 9""') avec un bras de réglage (26).

20. Collimateur et scanner selon la revendication 19, **caractérisé en ce que** le mouvement coulissant (13) oppose une force aux ressorts de rappel (27) au moyen des bras de réglage (26).

21. Collimateur et scanner selon l'une des revendications 1 à 20, **caractérisé en ce que** les surfaces latérales adjacentes 10 des diaphragmes (6, 6', 6" ou 7, 7', 7", 7'" ou 8, 8', 8", 8"', 8"" ou 9, 9', 9", 9"', 9"", 9""') s'étendent de sorte qu'une fente (28) conditionnée par des tolérances ne s'étend pas parallèlement à la trajectoire de rayon.

22. Collimateur et scanner selon la revendication 21, **caractérisé en ce que** les surfaces latérales (10) présentent dans la direction de coulissement des déports complémentaires s'étendant dans la direction de coulissement par rapport à la planéité des surfaces latérales (10).

23. Collimateur et scanner selon la revendication 21, **caractérisé en ce que** le diaphragme iris (5) est basculé par rapport à un plan de diaphragme (29) se trouvant en angle droit par rapport à l'axe optique de sorte qu'un rayon (2) ne peut pas être incident à travers une fente (28).

24. Collimateur et scanner selon l'une des revendications 1 à 23, **caractérisé en ce qu'**outre le diaphragme iris (5) il se trouve un diaphragme fixe (30) pour une protection supplémentaire dans la trajectoire de rayon dont l'ouverture est adaptée à l'ouverture la plus grande possible (12) du diaphragme iris (5).
